# EUROPÄISCHE PATENTSCHRIFT

(11) **EP 1 584 352 B1**
(45) Veröffentlichungstag und Bekanntmachung des Hinweises auf die Patenterteilung: **20.04.2011**
(21) Anmeldenummer: 05450042.6
(22) Anmeldetag: 08.03.2005
(51) Int. Cl.: A61N 2/02

(54) **Gerät zum Erzeugen elektromagnetischer Wechselfelder**
Device for generating electromagnetic fields
Appareil de génération des champs electromagnétiques

(30) Priorität: 06.04.2004 AT 6042004
(43) Veröffentlichungstag der Anmeldung: 12.10.2005
(73) Patentinhaber: Horst, Leopold, Ing., 4060 Leonding (AT)
(72) Erfinder: Horst, Leopold, Ing., 4060 Leonding (AT)
(74) Vertreter: Hübscher, Helmut

(56) Entgegenhaltungen:
- WO-A-96/11723
- AT-B- 409 823
- DE-A1- 10 157 024
- US-A- 5 314 401
- US-A1- 2002 052 634

## Beschreibung

Die Erfindung bezieht sich auf ein elektromedizinisches Gerät zum Erzeugen elektromagnetischer Wechselfelder mit einem über eine Steuereinrichtung ansteuerbaren Oszillator, mit einem an die Steuereinrichtung angeschlossenen Programmspeicher für Steuerprogramme zum Ansteuern des Oszillators für in einer Behandlungsfolge mit zeitlichem Abstand aufeinanderfolgende Behandlungen mit einem Zähler für die aufeinanderfolgenden Behandlungen und mit einer Anzeige- und Eingabeeinrichtung.

Um bestimmte therapeutische Wirkungen zu erzielen, ist es bekannt (AT 409 823 B), den menschlichen Körper in einer Folge von Behandlungen dem Einfluß elektromagnetischer Wechselfelder auszusetzen, deren Feldstärke und Schwingungsfrequenz in Abhängigkeit von der jeweiligen Behandlungsfolge vorgegeben werden, um beispielsweise die Feldstärke während einer Behandlungsfolge zunächst von Behandlung zu Behandlung zu steigern und dann gegen das Ende der Behandlungsfolge hin wieder zu verringern. Um trotz der notwendigen Anpassung dieser von der Behandlungsfolge abhängigen Parameter eine einfache Handhabung ohne besondere Vorkenntnisse sicherstellen zu können, ist eine an einen Zähler für die Anzahl der Behandlungen in einer Behandlungsfolge angeschlossene Steuereinrichtung vorgesehen, die mit einem Programmspeicher für Steuerprogramme zur Vorgabe der Frequenz, der Feldstärke und/oder der Laufzeit in Abhängigkeit vom Zählstand des Zählers in Verbindung steht, so daß durch die Steuerprogramme die für jede Behandlung in einer Behandlungsfolge erforderlichen Parameter bezüglich der Frequenz, der Feldstärke und/oder der Laufzeit vorab festgelegt werden. Diese bekannten Geräte lassen jedoch die therapeutische Wirkung der aufeinanderfolgenden Behandlungen unberücksichtigt, so daß unter Umständen eine nicht zielführende Behandlung über die vorgesehene Behandlungsfolge fortgesetzt wird, wenn die Behandlungen nicht unter einer ständigen ärztlichen Aufsicht vorgenommen werden. Es wurde zwar bereits vorgeschlagen, die Feldparameter des elektromagnetischen Wechselfeldes in Abhängigkeit von der Hautfeuchtigkeit und der Pulsfrequenz des Behandelten zu steuern, doch besteht bei einer solchen von Körperparametern abhängigen Steuerung der elektromagnetischen Wechselfelder die Gefahr, daß aufgrund eines unbewußten Reaktionsverhaltens des Menschen die überwachten Körperparameter nicht als Maß für eine Optimierung der angestrebten therapeutischen Wirkung der elektromagnetischen Wechselfelder gewertet werden können.

Der Erfindung liegt somit die Aufgabe zugrunde, ein elektromedizinisches Gerät zum Erzeugen elektromagnetischer Wechselfelder der eingangs geschilderten Art so auszugestalten, daß die subjektive Wirkung der aufeinanderfolgenden Behandlungen einfach erfaßt und gegebenenfalls zum Eingriff in die Steuerprogramme für die einzelnen Behandlungen genützt werden kann.

Die Erfindung löst die gestellte Aufgabe dadurch, daß die Steuereinrichtung eine die Ansteuerung des Oszillators in Abhängigkeit vom Zählstand des Zählers sperrende Schaltstufe, eine Anzeige- und Eingabeeinrichtung zur Anzeige eines vorgegebenen Datensatzes und zur Eingabe von aus dem Datensatz ausgewählter Daten sowie eine die Schaltstufensperre in Abhängigkeit von den ausgewählten Daten lösende Auswerteschaltung umfaßt.

Da zufolge dieser Maßnahmen nach einer vorgegeben Anzahl von Behandlungen die Weiterbehandlung von den Informationen abhängig gemacht werden kann, die über die Anzeige- und Eingabeeinrichtung durch die Auswahl bestimmter Daten aus vorgegebenen Datensätzen der Auswerteschaltung vorgelegt werden, kann die subjektive Bewertung des Behandlungserfolges in einfacher Weise erfaßt werden, um über die Auswerteschaltung programmbedingt zu entscheiden, ob aufgrund des festgestellten Behandlungserfolges die Behandlungsfolge entsprechend dem jeweiligen Steuerprogramm fortgesetzt werden soll. Zu diesem Zweck kann der Datensatz zu vorgegebenen Fragen standardisierte Antworten enthalten, die zusammen mit den Fragen angezeigt und entsprechend der subjektiven Bewertung des Behandlungserfolges ausgewählt werden. Wird über die Auswerteschaltung festgestellt, daß die vom Behandelten erfahrenen Wirkungen im Rahmen eines vorgesehenen Therapieverlaufes liegen, so wird die Schaltstufensperre über die Auswerteschaltung aufgehoben und das Gerät für eine weitere Anzahl von Behandlungen freigegeben, um nach dieser Behandlungsreihe gegebenenfalls eine weitere Abfrage vorzusehen. Entspricht der subjektiv erfahrene Behandlungserfolg nicht dem erwarteten Therapieverlauf, so sperrt die Schaltstufe die Ansteuerung des Oszillators über die Steuereinrichtung, um beispielsweise das zur Anwendung kommende Steuerprogramm hinsichtlich seiner Eignung für die Behandlung des Patienten ärztlich zu überprüfen. Durch eine Anzeige kann auf eine solche notwendige Überprüfung hingewiesen werden.

Damit die Fortführung der Behandlungen im Rahmen der vorgesehenen Behandlungsfolge nicht nur von einer einzigen Bewertung des behandelten Patienten abhängig gemacht wird, empfiehlt es sich, die Ansteuerung des Oszillators in Abhängigkeit vom Zählstand des Zählers wiederholt durch die Schaltstufe zu sperren. Wird die Auswerteschaltung in diesem Zusammenhang an einen Datenspeicher für die über die Anzeige- und Eingabeeinrichtung eingegebenen Daten angeschlossen, so stehen für die Beurteilung des Behandlungserfolges die subjektiven Bewertungen des Patienten über mehrere Behandlungsperioden zur Verfügung, was ein genaueres Bild über die jeweilige therapeutische Wirkung ergibt.

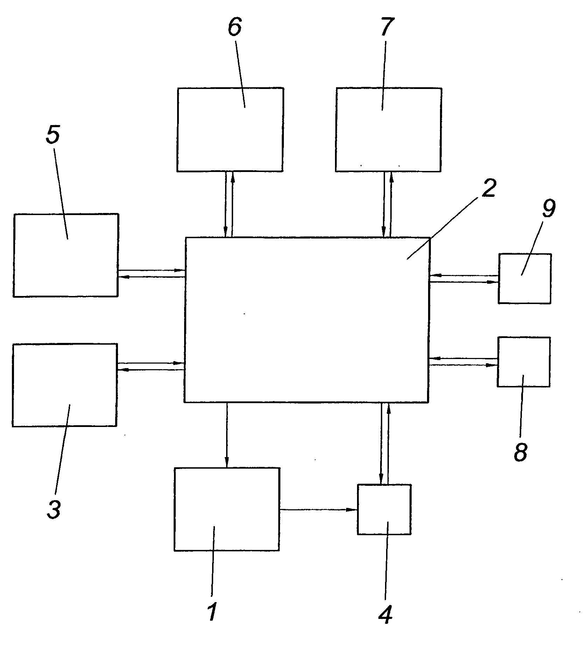

In der Zeichnung ist der Erfindungsgegenstand beispielsweise dargestellt, und zwar wird ein elektromedizinisches Gerät zum Erzeugen elektromagnetischer Wechselfelder in einem vereinfachten Blockschaltbild gezeigt.

Gemäß dem dargestellten Blockschaltbild weist das elektromedizinische Gerät einen Oszillator 1 zum Erregen eines elektromagnetischen Wechselfeldes über an den Oszillator angeschlossene elektrische Spulen auf. Dieser Oszillator 1 wird über eine Steuereinrichtung 2 angesteuert, mit deren Hilfe die Frequenz und Feldstärke des elektromagnetischen Feldes sowie die Laufzeit je Behandlung gesteuert wird. Die für die einzelnen Behandlungen in einer Behandlungsfolge vorgesehenen Parameter hinsichtlich der Frequenz, der Feldstärke und der Laufzeit werden über ein Steuerprogramm zur Verfügung gestellt, das aus einem Programmspeicher 3 ausgelesen wird. Da die einzelnen Behandlungen in einer vorgegebenen Behandlungsfolge unterschiedliche Parameter für die Ansteuerung des Oszillators 1 erfordern, werden die aufeinanderfolgenden Behandlungen gezählt. Zu diesem Zweck ist ein Zähler 4 vorgesehen, der mit jeder Laufzeit des Oszillators 1 angestoßen wird und seinen Zählstand um eins erhöht, so daß über die Steuereinrichtung 2 die jeweils vorzugebenden Parameter in Abhängigkeit vom Zählstand des Zählers 4 aus dem abgerufenen Steuerprogramm für die Ansteuerung des Oszillators 1 bereitgestellt werden können.

Damit der Behandlungserfolg nach einer vorgegebenen Anzahl von Behandlungen berücksichtigt werden kann, ist eine Schaltstufe 5 vorgesehen, die die Ansteuerung des Oszillators 1 in Abhängigkeit vom Zählstand des Zählers 4 sperrt. Diese Schaltstufensperre kann mit Hilfe einer Auswerteschaltung 6 nur dann aufgehoben werden, wenn eine subjektive Bewertung des Behandlungserfolges durch den behandelten Patienten vorliegt. Zu diesem Zweck ist eine Anzeige- und Eingabeeinrichtung 7 vorgesehen, über die ein aus einem Datensatzspeicher 8 ausgelesener Datensatz mit der Aufforderung zur Datenauswahl angezeigt wird. Dieser Datensatz enthält standardisierte Antworten auf ebenfalls angezeigte Fragen, die sich auf den Behandlungserfolg beziehen. Mit der Auswahl bestimmter Daten aus dem angezeigten Datensatz wird die Auswerteschaltung 6 beaufschlagt, um nach einem programmbedingten Vergleich der ausgewählten Daten mit einem vorgegebenen Therapieverlauf über die Freigabe der Schaltstufensperre zu entscheiden. Zeigen die über die Anzeige-und Eingabeeinrichtung 7 ausgewählten Daten einen entsprechenden Behandlungserfolg an, so wird die Ansteuerung des Oszillators 1 über die Steuereinrichtung 2 für eine weitere Anzahl von Behandlungen freigegeben. Die ausgewählten Daten werden in einem Datenspeicher 9 abgelegt, um diese Daten für eine Gesamtbeurteilung des Behandlungserfolges über mehrere Behandlungsperioden zur Verfügung zu haben. Wird durch die Auswerteschaltung 6 aufgrund der ausgewählten Daten ein nicht ausreichender Behandlungserfolg festgestellt, so wird dies in der Anzeige- und Eingabeeinrichtung 7 angezeigt, und zwar mit der Aufforderung, die Behandlung mit den elektromagnetischen Wechselfeldern überprüfen zu lassen. Ein Fachmann, im allgemeinen ein Arzt, kann dann in die Behandlungsfolge eingreifen und beispielsweise die Steuerparameter ändern.

Durch die geschilderten Maßnahmen wird es daher möglich, einem Patienten die Behandlung mit elektromagnetischen Wechselfeldern unter einer Überprüfung des Behandlungserfolges ohne ständige ärztliche Aufsicht angedeihen zu lassen.

## Patentansprüche

1. Elektromedizinisches Gerät zum Erzeugen elektromagnetischer Wechselfelder mit einem über eine Steuereinrichtung (2) ansteuerbaren Oszillator (1), mit einem an die Steuereinrichtung angeschlossenen Programmspeicher (3) für Steuerprogramme zum Ansteuern des Oszillators für in einer Behandlungsfolge mit zeitlichem Abstand aufeinanderfolgende Behandlungen, mit einem Zähler (4) für die aufeinanderfolgenden Behandlungen und mit einer Anzeige- und Eingabeeinrichtung (7), **dadurch gekennzeichnet, daß** die Steuereinrichtung (2) eine die Ansteuerung des Oszillators (1) in Abhängigkeit vom Zählstand des Zählers (4) sperrende Schaltstufe (5), eine Anzeige- und Eingabeeinrichtung (7) zur Anzeige eines vorgegebenen Datensatzes und zur Eingabe von aus dem Datensatz ausgewählter Daten sowie eine die Schaltstufensperre in Abhängigkeit von den ausgewählten Daten lösende Auswerteschaltung (6) umfaßt.

2. Elektromedizinisches Gerät nach Anspruch 1, **dadurch gekennzeichnet, daß** die Schaltstufe (5) die Ansteuerung des Oszillators (1) in Abhängigkeit vom Zählstand des Zählers (4) wiederholt sperrt.

3. Elektromedizinisches Gerät nach Anspruch 1 oder 2, **dadurch gekennzeichnet, daß** die Auswerteschaltung (6) an einen Datenspeicher (9) für die über die Anzeige- und Eingabeeinrichtung (7) eingegebenen Daten angeschlossen ist.

## Claims

1. Electro-medical device for generating alternating electromagnetic fields, having an oscillator (1) which can be actuated via a control device (2), having - connected to the control device - a program memory (3) for control programs for actuating the oscillator for treatments which succeed each other in a treatment sequence at spaced intervals of time, having a counter (4) for the successive treatments, and having a display and input device (7), **characterised in that** the control device (2) includes a switching step (5) blocking the actuation of the oscillator (1) in dependence upon the count on the counter (4), a display and input device (7) for displaying a preset data set and for inputting data selected from the data set, and an evaluation circuit (6) which releases the switching step block in dependence upon the selected data.

2. Electro-medical device as claimed in claim 1, **characterised in that** the switching step (5) repeatedly blocks the actuation of the oscillator (1) in dependence upon the count on the counter (4).

3. Electro-medical device as claimed in claim 1 or 2, **characterised in that** the evaluation circuit (6) is connected to a data memory (9) for the data input via the display and input device (7).

## Revendications

1. Appareil électromédical de génération de champs alternatifs électromagnétiques, avec un oscillateur (1), susceptible d'être commandé par l'intermédiaire d'un dispositif de commande (2), avec une mémoire à programmes (3), raccordée au dispositif de commande, pour des programmes de commande pour la commande de l'oscillateur pour des traitements se suivant les uns les autres avec un intervalle temporel dans une succession de traitements, avec un compteur (4) pour les traitements se suivant les uns les autres et avec un dispositif d'affichage et d'introduction (7), **caractérisé en ce que** le dispositif de commande (2) comprend un étage de commutation (5), bloquant la commande de l'oscillateur (1) en fonction de l'état de comptage du compteur (4), un dispositif d'affichage et d'introduction (7), pour l'affichage d'un jeu de données prédéterminé et pour l'introduction de données sélectionnées à partir du jeu de données, ainsi qu'un circuit d'évaluation (6), annulant le blocage de l'étage de commutation en fonction des données sélectionnées.

2. Appareil électromédical selon la revendication 1, **caractérisé en ce que** l'étage de commutation (5) bloque de manière répétée la commande de l'oscillateur (1) en fonction de l'état de comptage du compteur (4).

3. Appareil électromédical selon la revendication 1 ou 2, **caractérisé en ce que** le circuit d'évaluation (6) est raccordé à une mémoire de données (9) pour les données introduites par l'intermédiaire du dispositif d'affichage et d'introduction (7).
